# EUROPÄISCHE PATENTSCHRIFT

(11) **EP 0 979 087 B1**
(45) Veröffentlichungstag und Bekanntmachung des Hinweises auf die Patenterteilung: **12.11.2003**
(21) Anmeldenummer: 98921419.2
(22) Anmeldetag: 02.04.1998
(51) Int. Cl.: A61K 31/60, A61K 31/19, A61P 25/04

(54) **ANALGETIKA-KOMBINATION**
ANALGESIC COMBINATION
ASSOCIATION D'ANALGESIQUES

(30) Priorität: 15.04.1997 DE 19715594
(43) Veröffentlichungstag der Anmeldung: 16.02.2000
(73) Patentinhaber: BAYER AG, 51368 Leverkusen (DE)
(72) Erfinder: NEUSER, Dieter, D-40764 Langenfeld (DE); FIERUS, Monika, D-51381 Leverkusen (DE); WIEHL, Wolfgang, D-50999 Köln (DE)
(86) Internationale Anmeldenummer: EP9801926
(87) Internationale Veröffentlichungsnummer: WO98046235

(56) Entgegenhaltungen:
- WO-A-94/03160
- WO-A-96/29986
- GB-A- 1 026 502
- US-A- 4 446 140
- US-A- 4 593 044
- US-A- 4 851 233
- US-A- 5 064 858
- US-A- 5 096 926
- US-A- 5 451 409
- US-A- 5 702 723
- CHEMICAL ABSTRACTS, vol. 102, no. 7, 1984 Columbus, Ohio, US; abstract no. 55856, SHIBATA ET AL: "Effects of Combinations of Some Anti-Inflammatory Drugs with Lidocaine HCl on Inflammatory Pain" XP002076707 & SHIBATA ET AL: SHIKA KISO IGAKKAI ZASSHI, Bd. 26, Nr. 3, 1984, Seiten 872-881,

## Beschreibung

Die vorliegende Erfindung betrifft oral applizierbare Arzneizubereitungen enthaltend eine fixe Kombination von mindestens einem lokal wirkenden Analgetikum mit schnellem Wirkungseintritt und mindestens einem systemisch wirkenden Analgetikum mit langanhaltender Wirkung.

Es sind bereits lokal wirkende Analgetika mit schnellem Wirkungseintritt bekannt, die z.B. in Form von Sprays oder Lutschtabletten angewendet werden können. Solche Lokalanästhetika zeigen ihre Wirkung bereits nach weniger als einer Minute, besitzen aber nur eine kurze Wirkungsdauer, so daß häufig nachmediziert werden muß, was eine Beeinträchtigung der Sicherheit und der "patienten compliance" bedeutet.

Als besonders interessante lokal wirkende Analgetika seien z.B. die Benzocaine genannt. Sie hemmen die Reizbildung und -leitung in Nerven durch die Blockierung des Natriumstroms.

Systemisch wirkende Analgetika wie z.B. NSAIDS, insbesondere Acetylsalicylsäure (ASS) stellen eine andere gebräuchliche Möglichkeit zur Schmerzlinderung dar. Mit diesen Analgetika wird die Empfindlichkeit der Nocizeptoren herabgesetzt und die Schmerzlinderung ist durch die Hemmung der Prostaglandinsynthese zu erklären. Bei den meisten dieser systemisch wirkendenden Analgetika wird die maximale Wirksamkeit erst nach ca. 1 - 2 Stunden erreicht.

Aus US 4 446 140 sind Zusammensetzungen zur Behandlung von Mundschmerz bekannt, die das Antitussivum Dextrometorphan in Kombination mit einem Analgetikum wie Aspirin, Acetaminophen, Indomethacin, Ibuprofen oder Naproxen oder mit einem Anästhetikum wie Benzocain oder Butacain enthalten.

Aus WO 96/29986 sind Mikrokapseln zur Behandlung von Husten und Halsschmerzen bekannt. Die Mikrokapseln-Schale umhüllt eine Kern-Zusammensetzung, die ein Antitussivum oder ein Anästhetikum wie Lidocain oder Benzocain enthält sowie zudem Analgetika, Decongestantien, Expectorantien und Antihistaminica enthalten kann.

Aus US 5 064 858 sind oral verabreichbare Zusammensetzungen zur Behandlung von Narkotika-Abhängigkeit oder altersbedingten Zuständen wie Tinitus und Alzheimersche Krankheit bekannt, die einen geschützten Komplex von Procain und einem komplexierenden Agens wie Acetylsalicylsäure enthalten.

Shibata et al., *Chemical Abstracts* **1984,** *102*(7), 55856 beschreiben die Wirkungen von Kombinationen einiger anti-inflammatorischer Wirkstoffe, u. a. Aspirin, mit Lidocain-Hydrochlorid auf inflammatorischen Schmerz.

Aufgabe der vorliegenden Erfindung ist die Befriedigung des seit langem bestehenden Bedürfnisses eine oral applizierbare Zubereitung anzubieten, welche in einfacher und sicherer Weise eine sofortige analgetische Wirkung mit einer langanhaltenden Wirkung verbindet.

Gegenstand der Erfindung sind oral applizierbare Zubereitung enthaltend eine fixe Kombination von mindestens einem lokal wirkenden Analgetikum mit schnellem Wirkungseintritt (Element A) mit mindestens einem systemisch wirkenden Analgetikum mit langanhaltender Wirkung (Element B) und so formuliert, dass Elemant A zuerst freigesetzt wird.

Gegenstand der Erfindung ist weiterhin ein Verfahren zur Herstellung einer erfindungsgemäßen Zubereitung, wobei man die fixe Kombination eines Wirkstoffes des Elementes A und eines Wirkstoffes des Elementes B gemeinsam mit üblichen Hilfs- und Trägerstoffen und gegebenenfalls weiteren verträglichen Wirkstoffen so in eine geeignete Applikationsform überführt, dass Elemant A zuerst freigesetzt wird.

Als lokal wirkende Analgetika (Element A) können alle Wirkstoffe verwendet werden die einen signifikanten Wirkungseintritt in einem Zeitraum von bis zu 10 Minuten, bevorzugt von 4 Minuten, insbesondere von 1 Minute und ganz besonders von 30 Sekunden zeigen.

Die lokal wirkenden Analgetika (Kombinationselement A) werden zweckmäßigerweise in Mengen von 0,5 bis 100 mg, vorzugsweise 1 bis 60 mg und insbesondere 2 bis 30 mg pro einzelner Applikationsform eingesetzt.

Die erfindungsgemäße Kombination kann ein oder mehrere Lokalanästhetika als Element A enthalten, z.B. 1, 2 oder 3. Von besonderem Interesse sind Kombinationen mit nur einer Verbindung des Elements A.

Die Wirkstoffe gemäß Element A sind weitgehend bekannt. Als besonders geeignet seien beispielhaft genannt, esterartige Lokalanästhetika wie Benzocain, Amethocain, Amylocain, Butacain, Butoxycain, Butylaminobenzoat, Chloroprocain, Clormecain, Cyclomethycain, Isobutamben, Meprylcain, Oxybuprocain, Procain, Propipocain, Proxymetacain, Tricain etc. Ebenso seien anilid-artige Lokalanästhetika wie Lidocain, Bupivacain, Butanilicain, Carticain, Cinchocain, Clibucain, Etidocain, Mepivacain, Oxethazain, Prilocain, Ropivacain, Ethyl p-piperidinoacetyl-aminobenzoat, Tolycain, Trimecain, Vadocain etc.

Auch sonstige Lokalanästhetika wie z.B. Pramoxin oder ätherische Öle wie Menthol oder Eucalyptus-Öl sind einsetzbar.

Die als Element B einsetzbaren systemisch wirkenden Analgetika sind ebenfalls weitgehend bekannt. Vorzugsweise seien nicht steroidale entzündungshemmende Mittel (NSAIDs) genannt wie z.B. Phenylessigsäure-Derivate wie Aceclofenac, Alclofenac, Bromfenac, Diclofenac, Fenclofenac etc., Arylessigsäure-Derivate wie Acemetacin, Amfenacsodium, Bendazac, Glucametacin, Oxametacin etc., Para-Aminophenol-Derivate wie Acetanilid etc., Propionsäure-Derivate wie Alminoprofen, Ibuprofen, Ketoprofen, Flurbiprofen, Naproxen, Oxaprozin, Salicylsäure-Derivate wie Acetylsalicylsäure (ASS), Aluminium-ASS und andere Salze, Diflunisal. Etersalat, Fosfosal, Salol, Salsalat, Salacetamid etc, Pyrazolon-Derivate wie Amidopyrine, Dipyrone etc., Oxicam-Derivate wie Droxicam, Isoxicam, Piroxicam etc., Phenylbutazone-Derivate wie Azapropazone, Bumadizone, Calcium, Oxyphenbutazon etc., Pyrano-indolessigsäure-Derivate wie Etodolac etc., Anthranilsäure-Derivate wie Glafenin, Na-Meclofenamat, Mefenaminsäure, Morniflumat etc., Indol-Derivate wie Indomethacin etc., Paracetamol und Paracetamol-Derivate und sonstige NSAIDs wie Anirolac, Benzpiperylon, Benzydaminhydrochlorid, Na-Butibufen, Chlorthenoxazin, Cinmetacin, Clonixin, Cloracetadol, Difenpiramid, Diproqualon, Etenzamid, Famprofazon, Flupirtinmaleat, Ibuproxam, Indoprofen, Isamfazon, Meloxicam, Metiazinsäure, Metifenazon, Nifenazon, Niflumsäure, Mimesulid, Pirazoloc, Pranoprofen, Proquazon, Protizinsäure, Ramifenazon etc.

Die systemisch wirkenden Analgetika des Elementes B werden erfindungsgemäß eingesetzt in Mengen von 5 bis 1500 mg, vorzugsweise 8 bis 1000 mg, insbesondere 10 bis 800 mg pro Darreichungsform.

Als Element A werden vorzugsweise schnell wirkende lokale Analgetika eingesetzt, deren optimale Wirkungsdauer 0,5 bis 120 Minuten, vorzugsweise 2 bis 60 Minuten, insbesondere 5 bis 30 Minuten andauert. Als Element B werden vorzugsweise systemische Analgetika verwendet, deren signifikante Wirkung nach 15 Minuten eintritt und bis zu 24 Stunden dauert, vorzugsweise solche, deren Wirkung nach 20 Minuten eintritt und bis zu 12 Stunden, insbesondere bis zu 8 Stunden dauert.

In einer besonderen Ausführungsform der erfindungsgemäßen Zubereitung werden die Elemente A und B so ausgewählt, dass die fixe Kombination eine Wirkungsdauer von 2 Minuten bis zu 12 Stunden besitzt.

Von besonderem Interesse sind erfindungsgemäße Kombinationen die als Element A ein esterartiges Lokalanästhetikum, insbesondere Benzocain enthalten und als Element B Propionsäure-Derivate oder Salicylsäure-Derivate enthalten, insbesondere ASS.

Bevorzugte systemische Analgetika sind solche die eine Wirkungsdauer von mindestens 3 Stunden besitzen.

Die erfindungsgemäße Kombination ist besonders geeignet zur Behandlung entzündlicher und/oder schmerzhafter Erkrankungen des Mundrachenraums, insbesondere zur Behandlung von Pharyngitis, Laryngitis, Tonsillitis, Stomatitis, Gingivitis unterschiedlicher Ätiologie. Die Verabreichung des erfindungsgemäßen Kombinationspräparates erfolgt zweckmäßigerweise oral.

Die Kombination kann in üblichen Formulierungen eingesetzt werden, wobei das Lokalanästhetikum zuerst freigesetzt werden soll und das systemisch wirkende Analgetikum gegebenenfalls auch in Depotform vorliegen kann. Als Beispiel solcher Zubereitungen seien genannt: Kernmantel-Tabletten, Lutsch-Dragees, Kaugummi, Hart-Karamel mit flüssigem, halbfestem oder festem Kern. Ihre Herstellung erfolgt nach üblichen Methoden unter Verwendung gängiger Hilfsstoffe.

### Beispiele

### Beispiel 1

### Beispielhaft sei eine Tablette mit folgender Zusammensetzung genannt:

### ASS-Kerntablette:

500 mg ASS werden mit 30 mg Ascorbinsäure, 75 mg Saccharose, 47 mg mikrokristalline Cellulose, 2 mg Saccharin (550 fach) und 6 ml Orangensaft-Aroma zu einer Tablette vom Gesamtgewicht 660 mg gepreßt. Diese Kerntabletten werden mit einem Benzocain enthaltenden Dragiersirup gleichmäßig dragiert, wobei insgesamt ca. 5 mg Benzocain und 602 mg Dragiersirup aufgetragen werden. Die vorstehende Tablette zeigt bereits 2 Minuten nach Einnahme ein deutliche analgetische Wirkung die über einen Zeitraum von mehr als 3 Stunden anhält.

### Beispiel 2

In Analogie zu Beispiel 1 wird eine Kerntablette mit 300 mg Naproxen umhüllt mit einem Dragiersirup der 500 mg Lidocain enthält. Diese Kombinationszubereitung zeigt einen Wirkungseintritt nach 2 Minuten und eine Wirkungsdauer von mehr als 6 Stunden.

## Patentansprüche

1. Oral applizierbare Zubereitung enthaltend eine fixe Kombination von mindestens einem lokal wirkenden Analgetikum mit schnellem Wirkungseintritt (Element A) mit mindestens einem systemisch wirkenden Analgetikum mit langanhaltender Wirkung (Element B) und so formuliert, dass Elemant A zuerst freigesetzt wird.

2. Zubereitung gemäß Anspruch 1, **dadurch gekennzeichnet, dass** als Element A ein Wirkstoff eingesetzt wird, der eine optimale Wirkungsdauer von 0,5 bis 120 Minuten zeigt und als Element B ein Wirkstoff eingesetzt wird, mit einer Wirkung von 15 Minuten bis zu 24 Stunden.

3. Zubereitung gemäß Anspruch 1, **dadurch gekennzeichnet, dass** die Elemente A und B so ausgewählt werden, dass die fixe Kombination eine Wirkungsdauer von 2 Minuten bis zu 12 Stunden besitzt.

4. Verfahren zur Herstellung einer Zubereitung gemäß Anspruch 1, **dadurch gekennzeichnet, dass** man die fixe Kombination eines Wirkstoffes des Elementes A und eines Wirkstoffes des Elementes B gemeinsam mit üblichen Hilfs- und Trägerstoffen und gegebenenfalls weiteren verträglichen Wirkstoffen so in eine geeignete Applikationsform überführt, dass Elemant A zuerst freigesetzt wird.

## Claims

1. Preparation which can be administered orally, contains a fixed combination of at least one locally acting analgesic with a rapid onset of action (element A) with at least one systemically acting analgesic with a sustained action (element B), and is formulated such that element A is released first.

2. Preparation according to Claim 1, **characterized in that** the active substance employed as element A shows an optimal duration of action of from 0.5 to 120 minutes, and the active substance employed as element B has an action of from 15 minutes up to 24 hours.

3. Preparation according to Claim 1, **characterized in that** elements A and B are selected so that the fixed combination has a duration of action of from 2 minutes up to 12 hours.

4. Process for producing a preparation according to Claim 1, **characterized in that** the fixed combination of an active substance of element A and an active substance of element B are converted together with conventional ancillary substances and carriers and, where appropriate, other compatible active substances into a suitable administration form such that element A is released first.

## Revendications

1. Préparation par voie orale contenant une association fixe d'au moins un analgésique local à action rapide (élément A) avec au moins un analgésique systémique à action prolongée (élément B), formulée de telle sorte que l'élément A soit libéré en premier lieu.

2. Préparation selon la revendication 1, **caractérisée en ce que** l'élément A utilisé est un principe actif qui a une durée d'action optimale de 0,5 à 120 minutes et l'élément B utilisé est un principe actif d'une durée d'action de 15 minutes à 24 heures.

3. Préparation selon la revendication 1, **caractérisée en ce que** les éléments A et B sont choisis de telle sorte que l'association fixe possède une durée d'action de 2 minutes à 12 heures.

4. Procédé de préparation d'une préparation selon la revendication 1, **caractérisé en ce que** l'association fixe d'un principe d'actif de l'élément A et d'un principe actif de l'élément B avec des adjuvants et excipients courants et, éventuellement, d'autres principes actifs tolérés est présentée dans une forme d'application adéquate telle que l'élément A soit libéré en premier lieu.
